# EUROPEAN PATENT APPLICATION

(11) **EP 4 079 749 A1**
(43) Date of publication of application: **26.10.2022**
(21) Application number: 20903940.3
(22) Date of filing: 20.07.2020
(51) Int. Cl.: C07K 5/10, C07K 5/113, A61K 8/64, A61Q 19/00

(54) **PEPTIDE INHIBITING ACTIVITY OF ARYL HYDROCARBON RECEPTOR AND COSMETIC COMPOSITION USING SAME**

(30) Priority: 17.12.2019 KR 20190168700
(71) Applicant: Supadelixir Inc., Chuncheon-si, Gangwon-do 24232 (KR)
(72) Inventor: HAHN, Jang Hee, Chuncheon-si Gangwon-do 24375 (KR); KIM, Min Seo, Chuncheon-si Gangwon-do 24377 (KR)
(74) Representative: González López-Menchero, Álvaro Luis
(86) International application number: PCT/KR2020/009499
(87) International publication number: WO 2021/125484

(57) **Abstract**

Disclosed is a peptide and a cosmetic composition using the same. The peptide inhibits the activity of an aryl hydrocarbon receptor (AhR), whereby the peptide may be used to alleviate skin troubles caused by fine dust.

## Description

### Technical Field

The present disclosure relates to a peptide inhibiting the activity of an aryl hydrocarbon receptor (AhR) and a cosmetic composition using the same.

### Background Art

Fine dust refers to particles each having a size of less than 10 µm. Recently, the frequency of events in which the concentration of fine dust in the air exceeds the reference control level has been increasing. Such fine dust is mainly composed of carbon particles and is largely attributable to emissions from automobiles and exhaust gas from factories. The carbon particles contained in fine dust combine with organic compounds such as polyaromatic hydrocarbons and easily bind to an aryl hydrocarbon receptor (AhR) in cells, thereby inducing the release of reactive oxygen species and an inflammatory response, which in turn causes skin troubles.

Specifically, the AhR is a type of transcription factor that exists in the cytoplasm and has a Helix-loop-helix motif at the N-terminus. In the inactive state, the AhR binds to proteins such as hsp90, p23, and XAP2 and exists in a complex state. However, when the AhR is activated upon binding to halogenated aromatic hydrocarbons and polycyclic aromatic hydrocarbons in fine dust, it translocates to the nucleus, heterodimerizes with Arnt (aryl hydrocarbon receptor nuclear translocator) to act as a transcription factor, and binds to specific DNA response elements termed AHRE(AhR-response element), DRE(Dioxin-response element), XRE(Xenobiotics-response element) in the regulatory regions, promoting expression of various genes located downstream thus causing expression of various proteins, resulting in an abnormal gene expression which can lead to cytotoxicity.

For this reason, suppressors of aryl hydrocarbon receptor (AhR) activity that inhibit the activity of the AhR have been developed to prevent and treat diseases as disclosed in literatures listed below.

### <Patent Literature>

Korean Patent Application Publication No. 10-2019-0113902 (published on October 8, 2019) "Aryl Hydrocarbon Receptor (AhR) Modulator Compounds"

However, the existing aryl hydrocarbon receptor suppressors are not free from side effects when used for the treatment of diseases because they are mostly chemically synthesized.

### Disclosure

### Technical Problem

The present disclosure has been made keeping in mind the problems occurring in the related art.

The objective of the present disclosure is to provide a peptide inhibiting the activity of an aryl hydrocarbon receptor (AhR), and a cosmetic composition using the same, the peptide and the cosmetic composition being used to alleviate skin troubles induced by fine dust particles.

### Technical Solution

The present disclosure is implemented by embodiments having the following constructions to accomplish the above objective.

According to one embodiment of the disclosure, there is provided a peptide having an amino acid sequence of one of SEQ ID NOs: 1 to 4.

According to another embodiment of the disclosure, the peptide reduces an activity of an aryl hydrocarbon receptor (AhR).

According to a further embodiment of the disclosure, the peptide is used to alleviate skin troubles caused by fine dust.

According to a yet further embodiment of the disclosure, the peptide inhibits inflammation caused by fine dust.

According to a yet further embodiment of the disclosure, there is provided a cosmetic composition including a peptide having the amino acid sequence of one of SEQ ID NOs: 1 to 4.

According to a yet further embodiment of the disclosure, the cosmetic composition may have an effect of alleviating skin troubles caused by fine dust.

### Advantageous Effects

According to the above-described embodiments, the present disclosure has advantages described below.

The present disclosure uses the peptide having the amino acid sequence of one of SEQ ID NOs: 1 to 4 to inhibit the activity of an aryl hydrocarbon receptor (AhR), thereby alleviating skin troubles caused by fine dust.

### Description of Drawings

FIG. 1 is a graph showing the effect of the peptide, according to an embodiment of the disclosure, on physical interaction between the AhR and POLII;
FIG. 2 is a graph showing the effect of the peptide, according to an embodiment of the disclosure, on cytotoxicity induced by fine dust; and
FIG. 3 is a graph showing the effect of the peptide, according to an embodiment of the disclosure, on the production of reactive oxygen species induced by fine dust.

### Best Mode

Hereinafter, a peptide inhibiting the activity of an aryl hydrocarbon receptor (AhR) and a cosmetic composition using the same according to preferred embodiments of the present disclosure will be described in detail with reference to the accompanying drawings. Unless not specifically defined, all terminologies in the specification should be interpreted based on the general meanings thereof that a person skilled in the art understands. When the general meanings of the terminologies are incompliant with those used in the specification, the terminologies should be interpreted as being defined herein. In describing the present disclosure, well-known functions or constructions will not be described in detail in case they may unnecessarily obscure the understanding of the present disclosure. Also, when a part "includes" or "comprises" an element in the description, unless there is a particular description contrary thereto, the part can further include other elements, not excluding the other elements.

In the present disclosure, an embodiment relates to a peptide inhibiting the activity of an aryl hydrocarbon receptor (AhR). The peptide contains the amino acid sequence of SEQ ID NO: 1 (Asp-Arg-Leu-Asn), 2 (Asp-Arg-Leu), 3 (Asn-Thr-Glu-Leu), or 4 (Asn-Thr-Glu). The peptide inhibits the activity of the AhR, thereby alleviating skin problems caused by fine dust binding to the AhR. Specifically, the peptide suppresses the AhR activity and inhibits the production of cytotoxicity, reactive oxygen species, and inflammatory cytokines induced by fine dust binding to the AhR, thus may alleviate skin troubles.

In the present disclosure, another embodiment relates to a cosmetic composition including a peptide consisting of an amino acid sequence of one of SEQ ID NOs: 1 to 4. The cosmetic composition inhibits the production of cytotoxicity, reactive oxygen species, and inflammatory cytokines thus may alleviate skin problems caused by fine dust.

The cosmetic composition may further include one or more components selected from the group consisting of water-soluble vitamins, oil-soluble vitamins, high molecular weight polysaccharides, sphingolipids, and seaweed extracts.

As for the water-soluble vitamins, anything that can be blended in a cosmetic can be used. Preferably, examples thereof include vitamin B1, vitamin B2, vitamin B6, pyridoxine, pyridoxine hydrochloride, vitamin B12, pantothenic acid, nicotinic acid, nicotinic acid amide, folic acid, vitamin C, and vitamin H. However, salts of the examples, such as thiamine hydrochloride, sodium ascorbate, and derivatives thereof, such as ascorbic acid-2-phosphate sodium salt, and ascorbic acid-2-phosphate magnesium salt may also be included in the examples of the water-soluble vitamins to be used in the embodiment. The water-soluble vitamins may be obtained by conventional methods such as microbial transformation, purification from microbial culture, an enzymatic method, and a synthesizing method.

As for the oil-soluble vitamins, anything that can be blended in a cosmetic can be used. Preferably, examples thereof include vitamin A, carotin, vitamin D2, vitamin D3, and vitamin E (d1-alpha tocopherol, d-alpha tocopherol, d-alpha tocopherol). However, derivatives thereof, such as ascorbine palmitate, ascorbine stearate, ascorbine dipalmitate, dl-alpha tocopherol acetate, nicotinic acid dl-alpha tocopherol vitamin E, DLpantothenyl alcohol, D-pantothenyl alcohol, and pantothenyl ethyl ether may also be included in the examples of the oil-soluble vitamins to be used in the embodiment. The oil-soluble vitamins may be obtained by conventional methods such as microbial transformation, purification from microbial culture, an enzymatic method, and a synthesizing method.

As for the high molecular weight polysaccharides, anything that can be blended in a cosmetic can be used. Preferably, examples thereof include hydroxyethyl cellulose, xanthan gum, sodium hyaluronate, and chondroitin sulfate or a salt form (sodium salt) thereof. For example, chondroitin sulfate or its salt form can be mainly extracted and purified from sources like mammals and fish.

As for the sphingolipids, anything that can be blended in a cosmetic can be used. Preferably, examples thereof include ceramide, phytosphingosine, and sphingoglycolipids. The sphingolipids may be obtained by purifying an extract of mammals, fish, shellfish, yeast, or plants, or by chemical synthesis.

As for the seaweed extracts, anything that can be blended in a cosmetic can be used. Preferably, examples thereof include brown algae extract, red algae extract, and green algae extract. However, their purified extracts such as calrageenan, arginic acid, sodium alginate, and potassium arginate may also be included as seaweed extracts to be used in the embodiment. The seaweed extracts can be extracted and purified from seaweeds by conventional methods.

The cosmetic composition in the present disclosure may include other ingredients that are commonly used for cosmetic compositions. For example, one or more ingredients selected from among oil and fat ingredients, moisturizing agents, emollients, surfactants, organic and inorganic pigments, organic powders, ultraviolet absorbers, preservatives, fungicides, antioxidants, plant extracts, pH adjusters, alcohols, pigments, fragrances, blood circulation promoters, cooling agents, antiperspirants, and purified water may be further included.

The oil and fat ingredients include an ester oil, a hydrocarbon oil, silicone grease, fluorine-based fats, animal fats, and plant fats. Examples of the ester oil include glyceryl tri(2-ethylhexanoate), cetyl 2-ethylhexanoate, isopropyl myristate, butyl myristate, isopropyl palmitate, ethyl stearate, octyl palmitate, isocetyl isostearate, butyl stearate, ethyl linoleate, isopropyl linoleate, ethyl oleate, isocetyl myristate, isostearyl myristate, isostearyl palmitate, octyldodecyl myristate, isocetyl isostearate, diethyl sebacate, diisopropyl adipate, isoalkyl neopentanoate, tri(caprylic acid, capric acid) glyceryl, trimethylolpropane 2-ethylhexanoate, trimethylol propane triisostearate, pentaelysitol tetra(2-ethylhexanoate), cetyl caprylate, decyl laurate, hexyl laurate, myristic acid decyl, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linoleate, isopropyl isostearate, cetostearyl 2-ethylhexanoate, stearyl 2-ethylhexanoate, hexyl isostearate, ethylene glycol dioctanoate, ethylene glycol dioleate, propylene glycol dicapric acid, propylene glycol di(caprylic, capric acid), propylene glycol dicaprylate, neopentyl glycol dicapric acid, neopentyl glycol dioctanoate, glyceryl tricaprylate, glyceryl triundecyl acid, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerol oleic acid ester, polyglycerol isostearic acid ester, triisocetyl citrate, triisoalkyl citrate, triisooctyl citrate, lauryl lactate, myristyl lactate, cetyl lactate, octyldecyl lactate, triethyl citrate, acetyl triethyl citrate, acetyl tributyl citrate, trioctyl citrate, diisostearyl malate, 2-ethylhexyl hydroxystearate, di2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytsteryl isostearate, phytsteryl oleate, 12-stealoylhydroxystearate isocetyl, 12-stealoylhydroxystearate stearate, and 12-stealoylhydroxystearate isostearyl. The hydrocarbon oil may include squalene, liquid paraffin, alpha-olefin oligomer, isoparaffin, ceresin, paraffin, liquid isoparaffin, polybudene, microcrystalline wax, and petrolatum. The silicone grease may include polymethylsilicone, methylphenylsilicone, methylcyclopolysiloxane, octamethylpolysiloxane, decamethylpolysiloxane, dodecamethylcyclosiloxane, dimethylsiloxane-methylcetyloxysiloxane copolymer, dimethylsiloxane-methylstealloxysiloxane copolymer, alkyl-modified silicone oil, and amino-modified silicone oil. The fluorine-based fats may include perfluoropolyether. The animal and plant fats may include avocado oil, almond oil, olive oil, sesame oil, rice bran oil, bird flower oil, soybean oil, corn oil, rapeseed oil, apricot kernel oil, palm kernel oil, palm oil, castor oil, sunflower oil, grape seed oil, cottonseed oil, palm oil, kukui nut oil, wheat germ oil, rice germ oil, shea butter, evening primrose oil, macadamia nut oil, meadowfoam oil, egg yolk oil, tallow, horse oil, mink oil, orange raffia oil, jojoba oil, candelilla wax, carnauba wax, liquid lanolin, and hydrogenated castor oil.

The moisturizing agents may include a water-soluble low-molecular moisturizer, a fat-soluble molecular moisturizer, a water-soluble polymer, and a fat-soluble polymer. The water-soluble low-molecular moisturizer may include serine, glutamine, sorbitol, mannitol, pyrrolidone sodium carboxylate, glycerin, propylene glycol, 1,3-butylene glycol, ethylene glycol, polyethylene glycol B (polymerization degree n = 2 or more), polypropylene glycol(polymerization degree n = 2 or more), polyglycerin B(polymerization degree n = 2 or more), lactic acid, and lactate. The fat-soluble molecular moisturizer may include cholesterol and cholesterol ester. The water-soluble polymer may include carboxyvinyl polymer, polyaspartate, tragacanth, xanthan gum, methyl cellulose, hydroxymethylcellulose, hydroxyethylcellulose, hydroxypropylcellulose, carboxymethylcellulose, water soluble chitin, chitosan and dextrin. The fat-soluble polymer may include polyvinylpyrrolidone-eicocene copolymer, polyvinylpyrrolidonehexadecene copolymer, nitrocellulose, dextrin fatty acid ester, and high molecular weight silicone.

The emollients may include long chain acyl glutamic acid cholesteryl ester, cholesteryl hydroxystearate, 12-hydroxystearic acid, stearic acid, rosin acid, and lanolin fatty acid cholesteryl ester.

The surfactants may include nonionic surfactants, anionic surfactants, cationic surfactants, amphoteric surfactants. The nonionic surfactants may include self-emulsifying glycerin monostearate, propylene glycol fatty acid ester, glycerin fatty acid ester, polyglycerol fatty acid ester, sorbitan fatty acid ester, POE(polyoxyethylene), sorbitan fatty acid ester, POE sorbitan fatty acid ester, POE glycerin fatty acid ester, POE alkyl ether, POE fatty acid ester, POE hydrogenated castor oil, POE castor oil, POEPOP(polyoxyethylene polyoxypropylene) copolymer, POEPOP alkyl ether, polyether-modified silicone, lauric acid alkanolamide, alkylamine oxide, and hydrogenated soybean phospholipid. The anionic surfactants may include fatty acid soap, alpha-acylsulfonate, alkylsulfonate, alkylallylsulfonate, alkylnaphthalenesulfonate, alkylsulfate, POE alkyl ether sulfate, alkylamide sulfate, alkyl phosphate, POE alkyl phosphate, alkylamide phosphate, alkyloylalkyltaurine salt, N-acylamino acid salt, POE alkyl ether carboxylate, alkyl sulfosuccinate, sodium alkyl sulfoacetate, acylated hydrolyzed collagen peptide salt, and perfluoroalkyl phosphate ester. The cationic surfactants may include alkyltrimethylammonium chloride, stearyltrimethylammonium chloride, stearyltrimethylammonium bromide, cetostearyltrimethylammonium chloride, distearyldimethylammonium chloride, stearyldimethylbenzylammonium chloride, behenyltrimethylammonium bromide, benzalkonium chloride, diethylaminoethyl stearate, stearic acid dimethylaminopropylamide, and lanolin derivative quaternary ammonium salt. The amphoteric surfactants may include carboxybetaine type, amidebetaine type, sulfobetaine type, hydroxysulfobetaine type, amidesulfobetaine type, phosphobetaine type, aminocarboxylate type, imidazoline derivative type, and amidamine type.

The organic and inorganic pigments may include: the inorganic pigments such as silicic acid, silicic anhydride, magnesium silicate, talc, sericite, mica, kaolin, bengala, clay, bentonite, titanium coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine blue, chromium oxide, chromium hydroxide, calamine and their compounds; and the organic pigments such as polyamide, polyester, polypropylene, polystyrene, polyurethane, vinyl resin, urea resin, phenolic resin, fluororesin, silicon resin, acrylic resin, melamine resin, epoxy resin, polycarbonate resin, styrene-divinylbenzene copolymer, silk powder, cellulose, CI pigment yellow, and CI pigment orange; and organic and inorganic composite pigments.

The organic powders may include: metal soap such as calcium stearate; alkyl phosphate metal salt such as sodium zinc cetylate, zinc laurylate and calcium laurylate; acylamino acid polyvalent metal salt such as N-lauroyl-Beta-alanine calcium, N-lauroyl-Beta-alanine zinc, and N-lauroyl glycine calcium; amidesulfonic acid polyvalent metal salt such as N-lauroyl-taurine calcium and N-palmitoyl-taurine calcium; N-acyl basic amino acid such as N-epsilon-lauroyl-L-lysine, N-epsilon-palmitoylizine, N-alpha-paritoylolnithine, N-alpha-lauroylarginine, and N-alphahydrogenated beef fatty acid acylarginine; N-acyl polypeptide such as N-lauroylglycylglycine; alpha-amino fatty acids such as alphaaminocaprylic acid and alpha-aminolauric acid; and polyethylene, polypropylene, nylon, polymethyl methacrylate, polystyrene, styrene-divinylbenzene copolymer, and ethylene tetrafluoride.

The ultraviolet absorbers may include: paraaminobenzoic acid, ethyl para-aminobenzoate, amyl para-aminobenzoate, octyl para-aminobenzoate, ethylene glycol salicylate, phenyl salicylate, octyl salicylate, benzyl salicylate, butylphenyl salicylate, homomentyl salicylate, benzyl cinnamate, paramethoxycinnamic acid-2-ethoxyethyl, octyl para methoxycinnamate, dipara-methoxycinnamic acid mono-2-ethylhexaneglyceryl, isopropyl paramethoxycinnamate, diisopropyl·diisopropyl-based cinnamic acid ester mixture, urocanic acid, ethyl urokanate, hydroxymethoxybenzophenone, hydroxymethoxybenzophenonesulfonic acid and their salt form; and dihydroxymethoxybenzophenone, sodium dihydroxymethoxybenzophenonedisulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, and 2-(2-hydroxy-5-methylphenyl)benzotriazole.

The fungicides may include hinokitiol, triclosan, trichlorohydroxydiphenyl ether, chlorhexidine gluconate, phenoxyethanol, resorcin, isopropylmethylphenol, azulene, salicylic acid, zinc phyllithione, benzalkonium chloride, photosensitizer No. 301, mononitroguarechol sodium, and undecyrenic acid.

The antioxidants may include butylhydroxyanisole, propyl gallic acid, and elisorbic acid.

The pH adjusters may include citric acid, sodium citrate, malic acid, sodium malate, fumaric acid, sodium fumarate, succinic acid, sodium succinate, sodium hydroxide, and sodium monohydrogen phosphate.

The alcohols may include a higher alcohol such as cetyl alcohol.

Blending ingredients are not limited to the ones mentioned above, and any above-mentioned ingredient may be used for blending within the scope of not damaging the purpose and effect of the present disclosure. However, it would be preferable that the ingredient be blended at a blending ratio of 0.01 to 5% by weight, more preferably 0.01 to 3% by weight relative to the total weight of the cosmetic composition.

The cosmetic composition of the embodiment may take the form of solutions, emulsions, or viscous mixtures.

The cosmetic composition of the embodiment includes the ingredients described above as active ingredients but may also include other ingredients that are commonly used for cosmetics. For example, adjuvants and carriers such as stabilizers, solubilizers, vitamins, pigments, and fragrances may be included.

The cosmetic composition of the embodiment may be produced in any form of formulations that is conventionally used in the art. For example, it may be formulated into emulsions, creams, foundations, lotions, serums, or hair cosmetics. Specifically, it may be formulated into skin lotion, skin softener, moisture lotion, nourishing lotion, massage cream, nourishing cream, hand cream, moisture cream, essence, a pack, soap, lotion, milk lotion, gel, ointment, a patch, cleansing foam, body cleanser, astringent, and a spray. When the formulation comes in the form of paste, cream, or gel, carriers may be used. Examples of the carrier include animal fiber, vegetable fiber, wax, paraffin, starch, tracanth cellulose derivative, polyethylene glycol, silicone, bentonite, silica, talc, and zinc oxide. When the formulation comes in the form of powder or spray, the carrier may be lactose, talc, silica, aluminum hydroxide, calcium silicate, or polyamide. Especially in the case of powder, propellants such as chlorofluorohydrocarbon, propane/butane or dimethyl ether may be added. When the formulation comes in the form of solution or emulsion, the carrier may be a solvent, a solvating agent, or an emulsifying agent. Namely, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol, or sorbitan fatty acid esters may be used. When the formulation comes in the form of suspension, liquid diluent such as water, ethanol, propylene glycol, suspending agent such as ethoxylated isostearyl alcohol, polyoxyethylene sorbitol ester, polyoxyethylene sorbitan ester, microcrystalline cellulose, aluminum metahydroxide, bentonite, agar, and tracanth may be used as carriers. When the formulation comes in the form of surfactant-containing cleansing, fatty alcohol sulfate, fatty alcohol ether sulfate, sulfosuccinic acid monoester, isethionate, imidazolinium derivative, methyltaurate, sarcosinate, fatty acid amide ether sulfate, alkylamidobetaine, fatty alcohol, fatty acid glyceride, fatty acid diethanolamide, vegetable oil, lanolin derivatives, and ethoxylated glycerol fatty acid esters may be used as carriers.

Hereinbelow, the present disclosure will be described in greater detail with reference to exemplary embodiments. However, these embodiments are given by way of illustration only, and not by way of limitation.

### <Example 1> Peptide Synthesis

The peptide having the amino acid sequence of SEQ ID NO: 1, 2, 3, or 4, stated in Table 1 below, was synthesized using the FMOC solid-phase method with an automatic peptide synthesizer (PeptrEX-R48, Peptron, Korea). The synthesized peptide was purified and analyzed by reversed-phase high-speed liquid chromatography (Prominence LC-20AB, Shimadzu, Japan) using a C18 RP column (Shiseido Capcell pak), and was identified using a mass spectrometer (HP 1100 Series LC/MSD, Hewlett-Packard, USA).

**[Table 1]**

| Peptide Name | SEQ ID NO: | Amino Acid Sequence |
|---|---|---|
| AhR Pep1 | SEQ ID NO: 1 | Asp-Arg-Leu-Asn |
| AhR Pep2 | SEQ ID NO: 2 | Asp-Arg-Leu |
| AhR Pep3 | SEQ ID NO: 3 | Asn-Thr-Glu-Leu |
| AhR Pep4 | SEQ ID NO: 4 | Asn-Thr-Glu |

### <Example 2> Confirm the peptide produced in Example 1 inhibits the activity of an aryl hydrocarbon receptor (AhR), thereby interfering with physical binding of the AhR and POLII

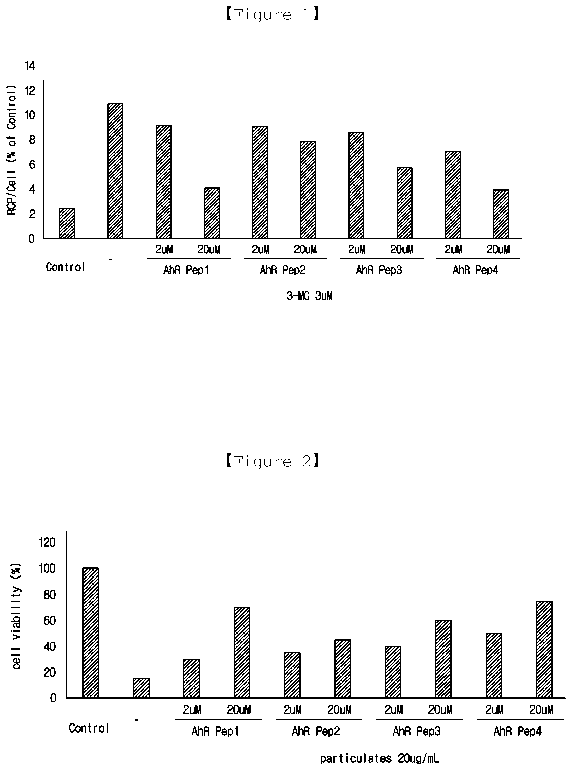

1. Human keratinocytes (HaCaT) were treated with the peptide produced in Example 1 and an aryl hydrocarbon receptor (AhR) antagonist 3-Methylcholanthrene (3-MC), then the effect of the peptide on physical interaction between the AhR and RNA polymerase II(POLII) was evaluated using in-situ PLA (proximity ligation assay).
2. Specifically, the HaCaT cells (4.5×10⁴ cells/well) were seeded into each well of a 24-well microplate in DMEM serum medium and cultured in an incubator at 37°C in the presence of 5% CO₂ for 24 hours. Then, the cultured cells were treated with 3-MC and the peptide produced in Example 1 (AhR Pep1 to AhR Pep4) respectively and incubated under the same conditions for 24 hours. In the medium, the concentration of 3-MC was 3 uM and the concentration of each peptide was 2 uM and 20 uM. As control groups, one with no treatment at all and one treated only with 3-MC were used. The cells in each well were washed with PBS, fixed in 2% formaldehyde for fifteen minutes, and treated with 0.1% TritonX-100 for five minutes to enhance antibody permeability into cells. Then, Anti-AhR polyclonal antibody (Abcam, UK) and Anti- POL**II** monoclonal antibody (Santa Cruz, USA) were added, PLA probe was applied using In situ PLA kit (Sigma-Aldrich), and steps of hybridization, ligation, amplification, and mounting were performed.
3. Luminescence signals (PLA signals) detected from each cell were measured with a confocal laser microscope (Olympus Fluoview FW1000; Olympus, Japan), then physical interaction between the AhR and POL**II** antibodies was quantified. The results are shown in FIG. 1.
4. As shown in FIG. 1, physical interaction between the AhR and POLII increases when human keratinocytes were treated with 3-MC, but when AhR Pep1 to AhR Pep4 were added with 3-MC, the increase in that interaction was reduced, thereby confirming that AhR Pep1 to AhR Pep4 inhibit the activity of an aryl hydrocarbon receptor (AhR). In other words, AhR Pep1 to AhR Pep4 inhibit the AhR activity, reducing the increase in interaction between the AhR and POL**II** by 3-MC, and in turn alleviating skin problems such as inflammation.

### <Example 3> Evaluate the cytotoxicity of the peptide produced in Example 1

1. MTT assay was performed to evaluate the cytotoxicity of the peptide produced in Example 1. The cultured HaCaT cells were treated with the peptide produced in Example 1 (AhR Pep1 to AhR Pep4) at a concentration of 0, 2, 20, and 50 nM respectively and cultured for 24 hours. Subsequently, an MTT solution was added and incubated, the resulting supernatant is removed and DMSO was added, then absorbance was measured at 540 nm.
2. As a result of the measurement, it was confirmed that AhR Pep1 to AhR Pep4 have no cytotoxicity.

### <Example 4> Assess the ability of the peptide produced in Example 1 to inhibit cytotoxicity induced by fine dust

1. To confirm that the peptide produced in Example 1 inhibits cytotoxicity induced by fine dust, the cultured HaCaT cells were treated with 20 ug/ml of fine dust (diesel particulate matter purchased from Sigma-Aldrich was used) and 2 uM and 20 uM of the peptide produced in Example 1 (AhR Pep1 to AhR Pep4) respectively, and incubated for 24 hours. Next, an MTT solution was added and further incubated, then DMSO was added and absorbance was measured at 540 nm. Finally, cell viability was calculated and the result is shown in FIG. 2. As control groups, one with no treatment at all and one treated only with fine dust were used.
2. As shown in FIG. 2, cell viability was significantly decreased when the cultured HaCaT cells were treated only with fine dust, whereas cell viability was significantly increased when AhR Pep1 to AhR Pep4 were added at the same time compared with being treated only with fine dust. Fine dust causes cytotoxic effects upon binding to the AhR. The peptide produced in Example 1, however, inhibits the binding of fine dust and the AhR, thereby reducing cytotoxicity production, and in turn alleviating skin problems such as inflammation.

### <Example 5> Assess the ability of the peptide produced in Example 1 to suppress the generation of reactive oxygen species by fine dust

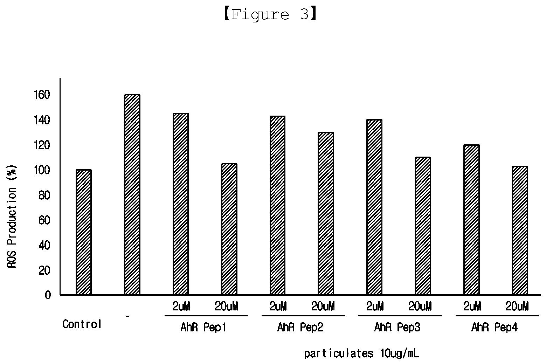

1. DCF-DA probe was used to confirm that the peptide produced in Example 1 suppresses the generation of reactive oxygen species by fine dust.
2. Specifically, the cultured HaCaT cells were treated with 10 ug/ml of fine dust and 2 uM and 20 uM of the peptide produced in Example 1 (AhR Pep1 to AhR Pep4) respectively, and incubated for 24 hours. Next, chlorofluorescein diacetate (DCFH-DA) was added for 30 minutes at 37°C, then the cells were analyzed using flow cytometer. The result is shown in FIG. 3.
3. As shown in FIG. 3, the generation of reactive oxygen species was significantly increased when the cultured HaCaT cells were treated only with fine dust, whereas the increase in the generation of reactive oxygen species by fine dust was suppressed when AhR Pep1 to AhR Pep4 were added at the same time. Fine dust generates reactive oxygen species upon binding to the AhR. The peptide produced in Example 1, however, inhibits the binding of fine dust and the AhR, thereby reducing the production of reactive oxygen species, and in turn contributing to alleviation of skin problems such as inflammation.

### <Example 6> Assess the ability of the peptide produced in Example 1 to inhibit the production of pro-inflammatory cytokines caused by fine dust

1. ELISA was used to confirm that the peptide produced in Example 1 inhibits the production of pro-inflammatory cytokines caused by fine dust.
2. Specifically, the cultured HaCaT cells were treated with 10 ug/ml of fine dust and 20 uM of the peptide produced in Example 1 (AhR Pep1 to AhR Pep4) respectively, and incubated for 24 hours. Then, IL-6 and TNF-α contents were measured using a mouse enzyme-linked immunosorbent assay (ELISA) kit (R&D Systems Inc, Minneapolis, MN, USA). The result is shown in Table 2.
3. As shown in Table 2, the production of cytokines was significantly increased when the cultured HaCaT cells were treated only with fine dust, whereas the increase in the production of cytokines by fine dust was suppressed when AhR Pep1 to AhR Pep4 were added at the same time. Fine dust generates cytokines upon binding to the AhR. The peptide produced in Example 1, however, inhibits the binding of fine dust and the AhR, thereby reducing cytokines production, and in turn contributing to alleviation of skin problems such as inflammation.

**[Table 2]**

| | No Treatme nt | Fine Dust | Fine Dust+ AhR Pep 1 | Fine Dust+ AhR Pep 2 | Fine Dust+ AhR Pep 3 | Fine Dust+ AhR Pep 4 |
|---|---|---|---|---|---|---|
| IL-6 product ion(%) | 0 | 100 | 76 | 87 | 83 | 74 |
| TNF-α product ion(%) | 0 | 100 | 79 | 93 | 89 | 82 |

Although the preferred embodiments have been described above, it is noted that such embodiments are presented only for illustrative purpose and thus any modifications or changes to the embodiments to implement the technical idea of the present disclosure should be construed to fall within the scope of the prevent invention.

## Claims

1. A peptide comprising an amino acid sequence of any one of SEQ ID NOs: 1 to 4.

2. The peptide of claim 1, wherein the peptide reduces an activity of an aryl hydrocarbon receptor (AhR).

3. The peptide of claim 1, wherein the peptide is used to alleviate skin troubles caused by fine dust.

4. The peptide of claim 3, wherein the peptide inhibits inflammation caused by fine dust.

5. A cosmetic composition comprising a peptide comprising an amino acid sequence of any one of SEQ ID NOs: 1 to 4.

6. The cosmetic composition of claim 5, wherein the cosmetic composition provides an effect of alleviating skin troubles caused by fine dust.


<110>    SUPADELIXIR INC.

<120>    Peptide for inhibiting activity of aryl hydrocarbon receptor and
         Cosmetic composition using the same

<130>    PFAHJ-20122

<150>    KR 10-2019-0168700
<151>    2019-12-17

<160>    4

<170>    KoPatentIn 3.0

<210>    1
<211>    4
<212>    PRT
<213>    Artificial Sequence

<220>
<223>    Peptide 1


<400>    1
Asp Arg Leu Asn
  1


<210>    2
<211>    3
<212>    PRT
<213>    Artificial Sequence

<220>
<223>    Peptide 2


<400>    2
Asp Arg Leu
  1


<210>    3
<211>    4
<212>    PRT
<213>    Artificial Sequence

<220>
<223>    Peptide 3


<400>    3
Asn Thr Glu Leu
  1


<210>    4
<211>    3
<212>    PRT
<213>    Artificial Sequence

<220>
<223>    Peptide 4

<400>   4
Asn Thr Glu
1